(19) Europäisches Patentamt / European Patent Office / Office européen des brevets

(11) **EP 3 741 300 A1**

(12) **EUROPEAN PATENT APPLICATION**
published in accordance with Art. 153(4) EPC

(43) Date of publication:
**25.11.2020 Bulletin 2020/48**

(21) Application number: **19793132.2**

(22) Date of filing: **25.04.2019**

(51) Int Cl.:
*A61B 5/1455* (2006.01)  *G01N 21/17* (2006.01)
*G01N 21/49* (2006.01)

(86) International application number:
**PCT/JP2019/017746**

(87) International publication number:
**WO 2019/208718 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2018 JP 2018085622**

(71) Applicant: **Medical Photonics Co., Ltd.**
**Sapporo-shi, Hokkaido 001-0021 (JP)**

(72) Inventor: **IINAGA, Kazuya**
**Sapporo-shi, Hokkaido 001-0021 (JP)**

(74) Representative: **Vossius & Partner**
**Patentanwälte Rechtsanwälte mbB**
**Siebertstrasse 3**
**81675 München (DE)**

(54) **LIPID CONCENTRATION MEASUREMENT DEVICE AND METHOD THEREFOR**

(57)  [Problem] To provide a device which reduces individual differences in lipid concentration measurements by non-invasive lipid measurements.

[Solution] The invention comprises: an irradiation unit irradiating a light of a predetermined light intensity onto an organism; a light intensity detection unit positioned at a predetermined distance from the irradiation unit and detecting the light intensity emitted from the organism; and a control unit calculating, on the basis of the light intensity, the blood flow turbulence intensity, and calculating, from the turbulence intensity, the lipid concentration.

FIG. 1

A = SKIN LAYER
B = SKIN LAYER + BLOOD LAYER
C = SKIN LAYER + BLOOD LAYER + MUSCLE LAYER

**Description**

Technical Field

The present invention relates to a device for measuring the concentration of in-blood lipid and a method therefor.

Background Art

Attention has been directed to postprandial hyperlipidemia as a risk factor of the arteriosclerosis. To diagnose the postprandial hyperlipidemia, it is necessary to observe a change in in-blood lipid concentration for 6 to 8 hours after meals. That is, to measure the state of postprandial hyperlipidemia, it is necessary to place a subject under restraint for 6 to 8 hours and collect blood multiple times. The diagnosis of the postprandial hyperlipidemia is therefore no better than clinical studies, and diagnosing the postprandial hyperlipidemia at a clinical site is not practical.
Patent Literature 1 discloses a method that allows measurement of in-blood lipid not only in a medical institution but at home by eliminating blood collection. Allowing instantaneous data acquisition allows temporally continuous in-blood lipid measurement.

Citation List

Patent Literature

Patent Literature 1: International Publication No. 2014/087825

Summary of Invention

Technical Problem

In the method described in Patent Literature 1, however, individual differences in a case where the measurement is performed on a plurality of persons result in different measured values in some cases, which is a problem in data sharing and reference value setting.
The present invention has been made to solve the problem with the related art, and an object of the present invention is to provide a device for reducing individual differences in lipid concentration measurement and a method therefor.

Solution to Problem

A lipid concentration measurement device according to the present invention includes a light radiator that radiates light having a predetermined optical intensity to a biological body, an optical intensity detector that is located at a predetermined distance from the light radiator and detects an optical intensity of light emitted from the biological body, and a control unit that calculates a turbulent flow intensity in a blood flow based on the optical intensity and calculates a lipid concentration based on the turbulent flow intensity.
A lipid concentration measurement device according to the present invention includes a light radiator that radiates light having a predetermined optical intensity to a biological body, optical intensity detectors that are arranged at predetermined intervals from the light radiator, continuously from the light radiator, or in front of the light radiator and detect optical intensities of light emitted from the biological body, and a control unit that calculates a light scatter coefficient in the biological body based on the optical intensities, calculates a turbulent flow intensity in a blood flow based on the scatter coefficient, and calculates a lipid concentration based on the turbulent flow intensity.
A lipid concentration measurement device according to the present invention is a lipid concentration measurement device communicably connected to a first device including a light radiator that radiates light having a predetermined optical intensity to a biological body, an optical intensity detector that is located at a predetermined distance from the light radiator and detects an optical intensity of light emitted from the biological body, and a communicator that transmits the optical intensity detected by the optical intensity detector, and the lipid concentration measurement device includes a control unit that calculates a turbulent flow intensity in a blood flow based on the optical intensity transmitted from the first device and calculates a lipid concentration based on the turbulent flow intensity.
A lipid concentration measurement device according to the present invention is a lipid concentration measurement device communicably connected to a first device including a light radiator that radiates light having a predetermined optical intensity to a biological body, optical intensity detectors that are arranged at predetermined intervals from the light radiator, continuously from the light radiator, or in front of the light radiator and detect optical intensities of light emitted from the biological body, and a communicator that transmits the optical intensities, and the lipid concentration measurement device includes a control unit that calculates a light scatter coefficient in the biological body based on the optical intensities transmitted from the first device, calculates a turbulent flow intensity in a blood flow based on the scatter coefficient, and calculates a lipid concentration based on the turbulent flow intensity.

A lipid concentration measurement method according to the present invention includes a light radiation step of radiating light having a predetermined optical intensity to a biological body, an optical intensity detection step of detecting an optical intensity of light emitted from the biological body located at a predetermined distance from a position to which the light is radiated in the light radiation step, a turbulent flow intensity calculation step of calculating a turbulent flow intensity in a blood flow based on the optical intensity, and a lipid concentration calculation step of calculating a lipid concentration based on the turbulent flow intensity.

A lipid concentration measurement method according to the present invention includes a light radiation step of radiating light having a predetermined optical intensity to a biological body, an optical intensity detection step of detecting optical intensities of light emitted from the biological body and measured at predetermined intervals from a position to which the light is radiated in the light radiation step, continuously from the position, or in front of the position, a scatter coefficient calculation step of calculating a light scatter coefficient in the biological body based on the optical intensities, a turbulent flow intensity calculation step of calculating a turbulent flow intensity in a blood flow based on the scatter coefficient, and a lipid concentration calculation step of calculating a lipid concentration based on the turbulent flow intensity.

Advantageous Effects of Invention

The lipid concentration measurement device according to the present invention and a method therefor allow reduction in individual differences in lipid concentration measurement.

Brief Description of Drawings

[Figure 1] Figure 1 shows the skin, blood, and muscle contained in the optical path from a light radiator to a light receiver through a biological body.
[Figure 2] Figure 2 shows measured individual differences among persons.
[Figure 3] Figure 3 shows the results of measurement of temporal changes in a blood scatter coefficient.
[Figure 4] Figure 4 shows a result of frequency analysis on noninvasive lipid continuous monitoring in a lipid load test.
[Figure 5] Figure 5 shows the results of measurement of temporal changes in the turbulent flow intensity.
[Figure 6] Figure 6 shows the correlation between the turbulent flow intensity and lipid concentration.
[Figure 7] Figure 7 shows the dependence of lipid particles on a change in TG in the lipid load test.
[Figure 8] Figure 8 shows the dependence of lipid particles on a change in TG in the lipid load test.
[Figure 9] Figure 9 shows the configuration of a lipid concentration measurement device according to a first embodiment.
[Figure 10] Figure 10 shows that in-blood lipid particles scatter light.
[Figure 11] Figure 11 shows an example in which an optical intensity detector is so disposed as to face a light radiator in the embodiment.
[Figure 12] Figure 12 shows an example in which the optical intensity detector is so disposed as to face the light radiator in the embodiment.
[Figure 13] Figure 13 shows the configuration of a control system of the lipid concentration measurement device according to the embodiment.
[Figure 14] Figure 14 shows the configuration of a lipid concentration measurement device according to a second embodiment.
[Figure 15] Figure 15 shows the amount of change in scatter coefficient μs' and the amount of change in average lipoprotein particle diameter.
[Figure 16] Figure 16 shows the configuration of a control system of the lipid concentration measurement device according to the embodiment.
[Figure 17] Figure 17 shows the configuration of a lipid concentration measurement device according to a third embodiment.
[Figure 18] Figure 18 shows the configuration of a lipid concentration measurement device according to a fourth embodiment.
[Figure 19] Figure 19 is a flowchart of a lipid concentration measurement method according to an embodiment.
[Figure 20] Figure 20 is a flowchart of the lipid concentration measurement method according to another embodiment.

Description of Embodiment

The measurement principle of a lipid concentration measurement device according to an embodiment and a method therefor will be described.

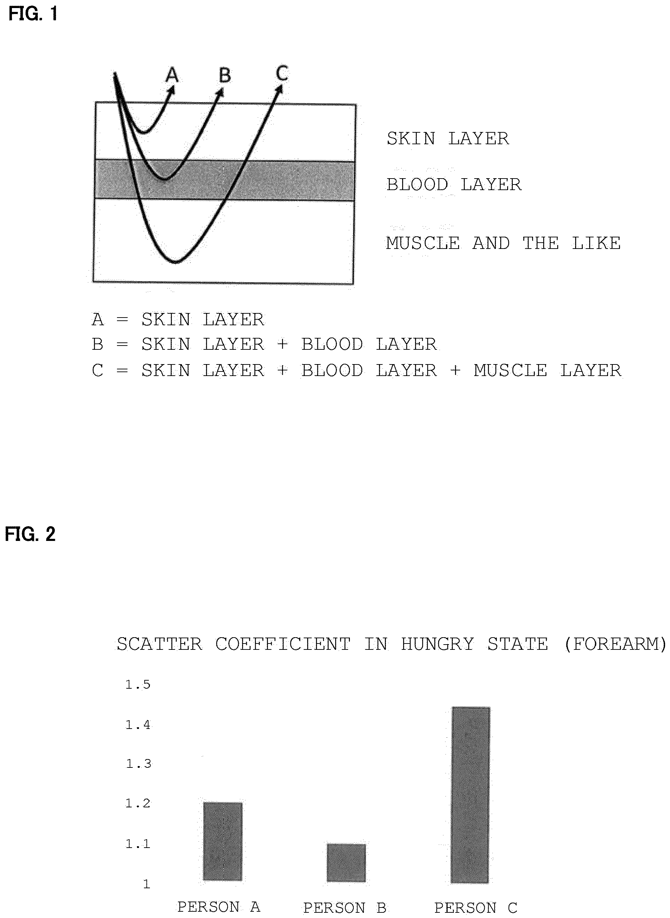

The results of measurement using light contain information on the skin, blood, muscle, and all other body parts contained in the optical path from a light radiator to a light receiver through the biological body, as shown in Figure 1. To obtain only information from the blood, it is necessary to remove information from the skin and muscle.

Figure 2 shows measured individual differences among persons. In actual noninvasive measurement, a difference of 0.1 (a.u.) in scattering intensity corresponds to a difference of about 150 mg/dL in TG (triglyceride) concentration as a converted value. Comparison among the current measurement results shows that the difference in TG concentration as a converted value between the persons B and C in Figure 2 is at least by 400 mg/dL. However, an analysis result after blood collection actually shows a difference of about only 20 mg/dL in TG concentration as a converted value.

The present inventor then focused on the fact that the blood keeps flowing in the actual biological body and tried to extract only blood information based on the blood motion.

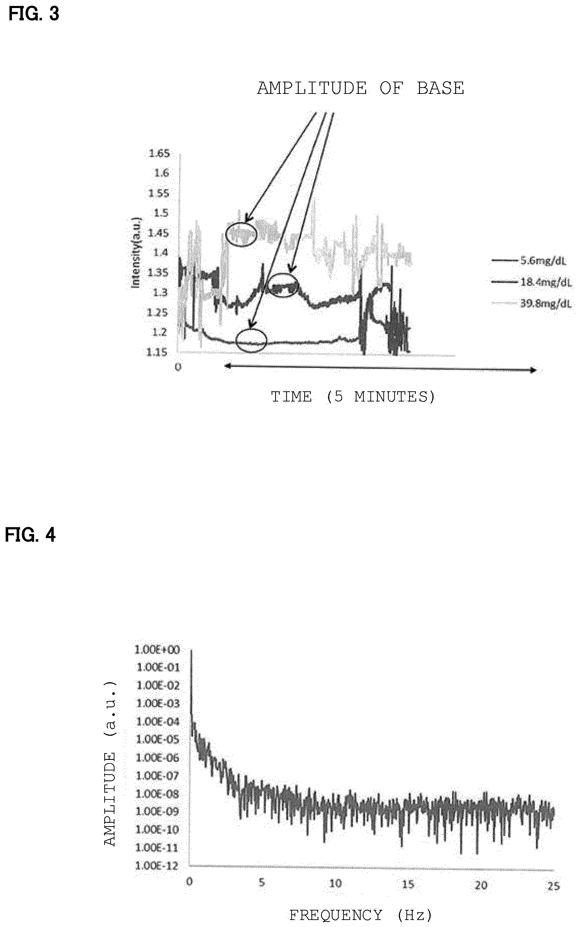

Figure 3 shows the results of measurement of temporal changes in blood scatter coefficient. Comparison between temporal changes in scatter coefficient (intensity along vertical axis) at different concentrations of chylomicron, which greatly affects optical quantities, shows that the higher the chylomicron concentration is, the wider the amplitude of the scatter coefficient is, as shown in Figure 3.

The chylomicron concentration in Figure 3 was determined by using the HPLC method. The average of the scatter coefficients over a fixed temporal range is used for the analysis in the prior technology, and a temporal change in the scatter coefficient is used for the analysis in the embodiment. Therefore, for comparison, the average over the fixed temporal range is shown as a base, and the variation in the scatter coefficient over the fixed period is shown as the amplitude of the base.

Figure 4 shows a result of frequency analysis using fast Fourier transform (FFT) performed on a result of noninvasive lipid continuous monitoring (six-hour measurement) in a lipid load test to locate a cause of the amplitude of the base.

No frequency characteristic was found in the lipid load test, as shown in Figure 4. That is, it can also be said that the amplitude of the base shown in Figure 3 is random noise.

That is, in the scatter measurement, periodic variation associated with the biological body, such as the heartbeat and respiration as representative examples, is not detected. The reason for this is that measurement at two or more points allows cancellation of the periodic variation associated with the blood.

Further, what is typically called vasomotion or blood flow fluctuation in a long cycle ranging from 0.1 to 0.2 Hz is observed in the blood flow measurement, whereas no vasomotion is observed in the scatter measurement. Also in this regard, a periodic change associated with the biological body is canceled.

The frequency characteristic shown in Figure 4 can be observed in one light receiver observation.

It is therefore believed that the amplitude shown in Figure 3 is an amplitude resulting from a random change in blood flow. Under this assumption, the present inventor focused on a turbulent blood flow.

It is believed that Reynold's number of the blood flowing through the body is about 2000, and the blood flow is typically a laminar flow. That is, the blood flow in the body can be taken as a stable, organized blood flow. On the other hand, a phenomenon in which the average diameter of the in-blood lipid particles increases occurs in the state in which the in-blood lipid concentration increases after a meal, so that the density of substances including lipid and the like contained in the blood increases.

Since Reynold's number increases as the density of substances in a fluid increases, it is believed that the blood flow after a meal has a large Reynold's number and the state of the blood flow changes from the laminar flow to the turbulent flow. It is said that a laminar flow changes to a turbulent flow when Reynold's number becomes greater than 2300, and the state of the blood flow is likely to change from a laminar flow to a turbulent flow and vice versa.

It is known that when the blood flow changes from a laminar flow to a turbulent flow, the blood cells and other blood components are deformed as the share rate increases. It is speculated that the shape of lipoprotein also randomly changes. That is, in optical scatter measurement, a change in the shape of lipoprotein particles due to a turbulent flow is measured in the form of fluctuation (turbulence) of the measured optical intensity.

Figure 3 shows the results of the lipid load test and can ascertain that the width between the maximum and the minimum of the amplitude of the base increases as the amount of chylomicron increases. It is believed that the reason for this is that an increase in the number of in-blood large particles, such as chylomicron particles, increases the intensity of the turbulent flow in the blood vessel. The turbulent flow intensity used in the specification refers to temporal fluctuation of the received light intensity resulting from the movement or motion of a substance under measurement.

A turbulent flow intensity I in the blood flow in a blood vessel can be determined as follows: Variation $\sigma S$ in the scatter coefficient in a sampling segment $\tau$ in noninvasive lipid measurement is determined by the expression below.

[Numerical expression 1]

$$\sigma S = \sqrt{S^2 + \overline{S}^2}$$

S : measured scatter coefficient in the measurement segment $\tau$
$\overline{S}$ : average of the scatter coeficients in the measurement segment $\tau$

The turbulent flow intensity I is determined by the expression below.

[Numerical expression 2]

$$I = \frac{\sigma S}{\overline{S}}$$

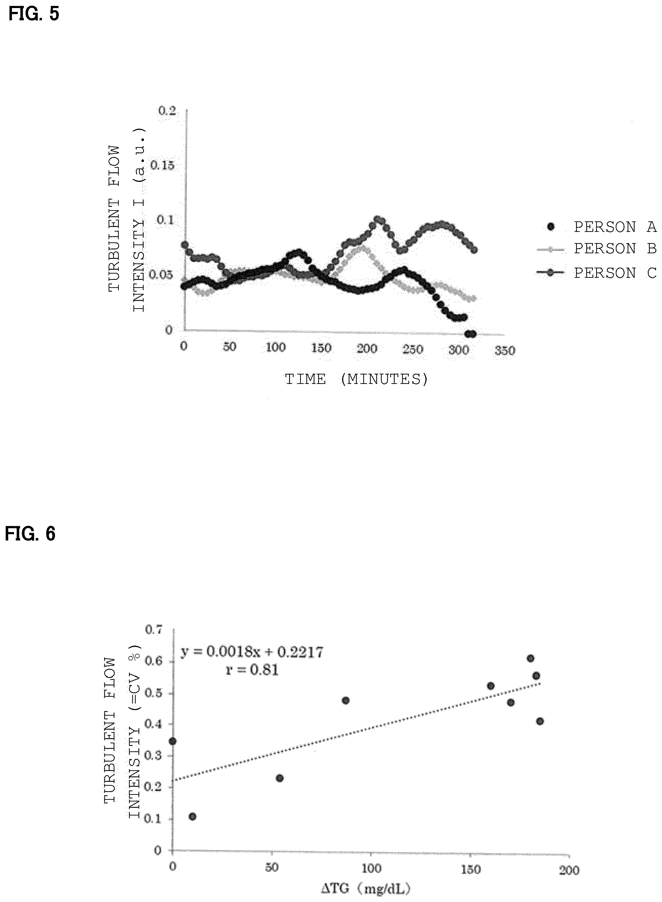

Figure 5 shows the results of measurement of the turbulent flow intensity I, that is, a temporal change in turbulence of the blood flow in the lipid load test. The TG concentrations in persons A, B, and C after blood collection were close to one another for the period from 50 to 100 minutes, as shown in Figure 5. It was therefore ascertained that the turbulent flow intensity I has been successfully measured in the segment from 50 to 100 minutes with no individual difference.

The turbulent flow intensity I can also be measured even in a simple one-point light reception. In the simple one-point light reception, the turbulent flow intensity I is calculated by the expression below. In the simple one-point light reception, the distance between the light radiator and the light receiver is preferably set at a value approximately from 1.5 to 2.0 cm.

The one-point measurement allows observation of the frequency characteristic shown in Figure 4, whereby the change in the frequency characteristic also allows derivation of the turbulent flow intensity I.

Also when the turbidity is determined by temporal resolution measurement using pulsed light, the light reception measurement can be performed only at one point. In this case, the sampling rate employed by the light receiver is more important than the cycle employed by the light source. For example, even when the data is acquired wirelessly, a change in blood turbidity can be measured as long as the sampling rate is at least 250 per second.

[Numerical expression 3]

$$X = \frac{1}{T\bar{x}} \int_0^T x d\theta$$

T: measurement period
x: measured optical intensity
$\bar{x}$: average of measured optical intensities
$\theta$ : period
X: turbulent flow intensity I

In actual measurement, the amplitude of the base (turbulence of measured value) only needs to be measured without distinction between a turbulent flow and a laminar flow.

Figure 6 shows the correlation between the turbulent flow intensity and the lipid concentration. Figure 6 shows that the correlation coefficient representing the correlation between the turbulent flow intensity and the lipid concentration is a satisfactory value of 0.81, and that an increase in lipid concentration increases the blood turbulent flow intensity.

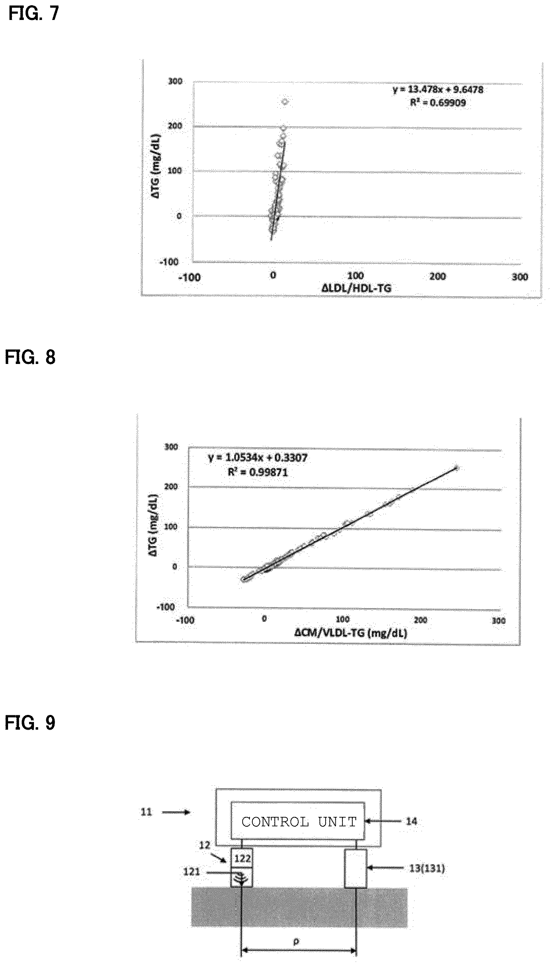

Figures 7 and 8 show the dependence of the lipid particles on the change in TG concentration in the lipid load test. The TG concentrations of HDL and LDL do not change at all, as shown in Figure 7. On the other hand, the TG concentrations in CM and VLDL change, as shown in Figure 8. It can therefore be ascertained that a change in TG concentration after a meal is a change in TG concentrations in CM and VLDL.

That is, TG, CM, and VLDL after a meal can be measured.

To further improve the correlation with the turbulent flow intensity, the accuracy of the correlation can also be improved by using information on the blood pressure, the pulsation, the amount of blood, and other factors.

As a method for evaluating variation in the optical intensity received for a fixed period, an evaluation index, such as a variation coefficient, may be used.

The lipid concentration measurement device and a method therefore that are the embodiment will next be described in detail with reference to the drawings.

Figure 9 shows the configuration of a lipid concentration measurement device according to a first embodiment.

A lipid concentration measurement device 11 includes a light radiator 12, an optical intensity detector 13, and a control unit 14, as shown in Figure 9.

The light radiator 12 includes a light source 122 for radiating radiated light to a predetermined radiation position 121 from a point outside the living body toward the interior of the living body. The light source 122 in the present embodiment can adjust the wavelength of the radiated light. The light source 122 can adjust the range of the wavelength in such a way that the wavelength range does not fall within the range of the wavelengths at which the light is absorbed by inorganic substances of the blood plasma. The light source 122 can perform the adjustment in such a way that the wavelength range does not fall within the range of the wavelengths at which the light is absorbed by the cell components of the blood. The cell components of the blood used herein are the red blood cells, white blood cells, and platelets in the blood. The inorganic substances of the blood plasma are water and electrolytes in the blood. The light source 122 is, for example, a fluorescent lamp, an LED, a laser, an incandescent lamp, an HID, or a halogen lamp. The illuminance of the light from the light source 122 may be controlled by the control unit 14 or a separately provided control circuit.

The light radiator 12 in the embodiment can arbitrarily adjust the temporal length of light radiation, for example, continuous light radiation or pulsed light radiation in accordance with a method for calculating the turbulent flow intensity, which will be described later. The light radiator 12 can arbitrarily modulate the intensity or phase of the radiated light.

The optical intensity detector 13 receives radiated light emitted from the living body toward the space outside the living body and detects the optical intensity of the radiated light. The optical intensity detector 13 may be a photodiode, a CCD, a CMOS device, or any other light receiving element.

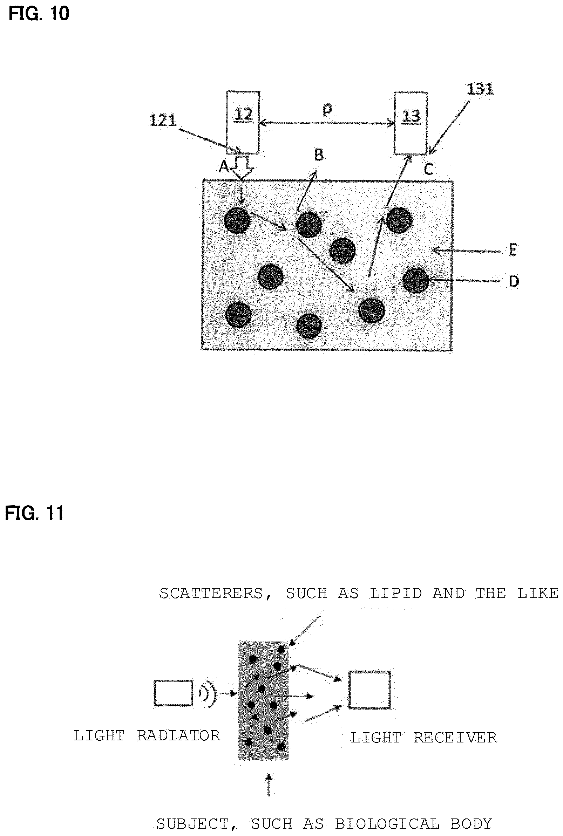

A predetermined distance $\rho$ is set between the radiation position 121, where the living body is radiated with the light, and a detection position 131, where the intensity of the light emitted from the blood (E in Figure 10) in the living body is detected, as shown in Figure 10. The thus set predetermined distance $\rho$ suppresses the influence of the light directly emitted from the living body (B in Figure 10), which is the radiated light (A in Figure 10) reflected off the surface of the living body and scatterers in the vicinity of the surface. The radiated light reaches the depth where lipid, such as lipoprotein, is present and is then reflected off the lipid (D in Figure 10) in the blood.

After the radiated light is reflectively scattered by the lipid, the optical intensity of the resultant back-scattered light (C in Figure 10) emitted from the living body is detected. Increasing the distance $\rho$ between the radiation position 121 and the detection position 131 increases the optical path length. The number of collisions between the light and the lipid therefore increases, so that the detected light is greatly affected by the scattering. Increasing the distance $\rho$ allows the influence of the scattering that is small and has therefore been difficult to be detected in related art to be readily captured.

Lipoprotein, which is the target under measurement, has a spherical structure covered with apoprotein and other substances. Lipoprotein is present in the form of a solid-like state in the blood. The lipoprotein is classified into CM, VLDL, LDL, and HDL in descending order of the particle diameter. Lipoprotein is characterized in that it reflects light. In particular, chylomicron (CM), VLDL, and other substances having a large particle diameter and specific gravity contain a large amount of triglyceride (TG) and are characterized in that they are more likely to scatter light. The optical intensity detected by the optical intensity detector 13 is therefore affected by the light scattered by lipoprotein.

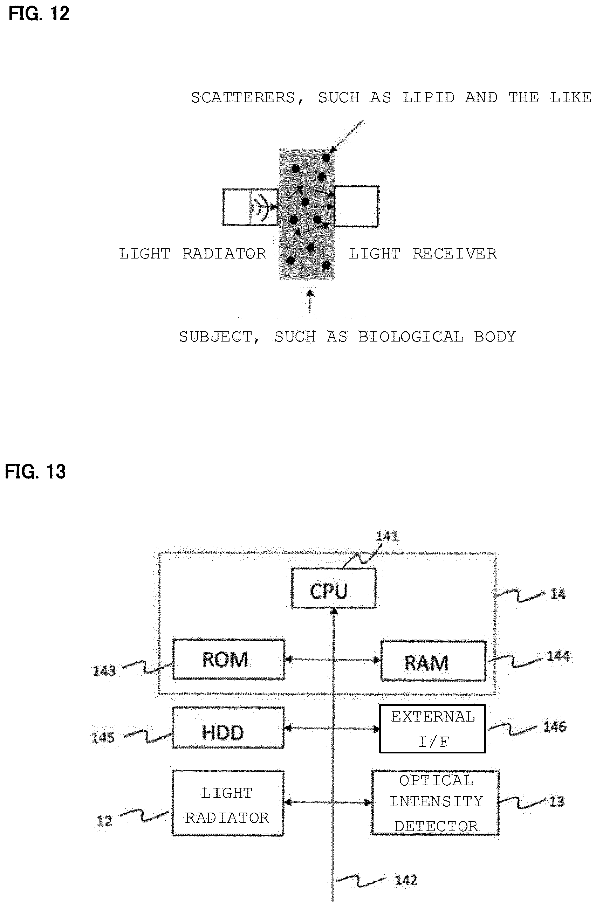

The optical intensity detector 13 may be so disposed as to face the light radiator 12, as shown in Figures 11 and 12. The arrangement can be employed to perform the measurement at a location where the thickness is not relatively thick and light is readily pass through a body part, such as an earlobe and a fingertip. The light radiator and the light receiver (optical intensity detector) may (Figure 12) or may not (Figure 11) be in contact with a subject under detection.

Figure 13 is a block diagram of the lipid concentration measurement device 11 according to the embodiment. A CPU (central processing unit) 141, a ROM (read only memory) 143, a RAM (random access memory) 144, a storage 145, an external I/F (interface) 146, the light radiator 12, and the optical intensity detector 13 are connected to each other via a system bus 142. The CPU 141, the ROM 143, and the RAM 144 form the control unit (controller) 14.

The ROM 143 stores in advance a program executed by the CPU 141 and thresholds used by the CPU 141.

The RAM 144 has an area where the program executed by the CPU 141 is developed, a variety of memory areas, such as a work area where the program processes data, and other areas.

The storage 145 stores data, such as sensed and calculated optical intensities. The storage 145 may be an internal memory that stores information in a nonvolatile manner, such as an HDD (hard disk drive), a flash memory, and an SSD (solid-state drive).

The external I/F 146 is an interface for communication with an external device, for example, a client terminal (PC). The external I/F 146 only needs to be an interface that performs data communication with the external device and may, for example, be an instrument (such as USB memory) locally connected to the external device or a network interface for the communication via a network.

The control unit 14 calculates the turbulent flow intensity in the blood flow based on the optical intensity detected by the optical intensity detector 13. In the embodiment, the optical measurement is performed by the simple one-point light reception. In the simple one-point light reception, the turbulent flow intensity I is calculated by Numerical Expression 4 below. In the simple one-point light reception, the distance ρ between the light radiator and the light receiver is preferably set at a value approximately greater than or equal to 1.5 cm but smaller than or equal to 2 cm.

Also when the turbidity is determined by temporal resolution measurement using pulsed light, one-point measurement can be performed. In this case, the sampling rate employed by the light receiver is more important than the cycle employed by the light source. For example, even when the data is acquired wirelessly, a change in blood turbidity can be measured as long as the sampling rate is at least 250 per second.

[Numerical expression 4]

$$X = \frac{1}{T\bar{x}} \int_0^T x\,d\theta$$

T:     measurement period
x:     measured optical intensity
x̄:     average of measured optical intensities
θ :    period
X:     turbulent flow intensity I

The control unit 14 calculates the in-blood lipid concentration based on the calculated turbulent flow intensity I.

Figure 6 shows the correlation between the turbulent flow intensity and the lipid concentration. Figure 6 shows that the turbulent flow intensity I (CV % along vertical axis of Figure 6) correlates with the lipid concentration (ΔTG mg/dL along horizontal axis of Figure 6). Figure 6 shows that the correlation coefficient is a satisfactory value of 0.81, and that an increase in lipid concentration increases the blood turbulent flow intensity. The lipid concentration can therefore be calculated based on the turbulent flow intensity I. In Figure 6, linear approximation is used, and any other approximation method, such as curve approximation, may be used as appropriate.

To further improve the correlation with the turbulent flow intensity, the accuracy of the correlation can also be improved by using information on the blood pressure, the pulsation, the amount of blood, and other factors.

In the embodiment, statistical data (or calibration curve) on the relationship between the turbulent flow intensity I and the amount of change in in-blood lipoprotein concentration, such as that shown in Figure 6, is collected in advance, and the collected data is stored in the storage 145. The measured turbulent flow intensity I is then compared with the stored statistical data or the calibration curve is used to calculate the amount of change in lipid concentration. The statistical data may be stored, for example, in the ROM 143.

The concentration and the turbidity can be used in the same meaning in a clinical site, and the concentration in the present embodiment also includes the concept of turbidity. Therefore, the result of the calculation performed by a lipid concentration calculator 5 can be not only the concentration but the number of particles per unit quantity, the formazin turbidity, or the amount of change in average lipid particle diameter.

The format of the statistical data is not limited to a specific format. For example, the statistical data may be classified in terms of gender, body height, body weight, BMI, or any other factor and may be calculated by using a table, a graph, a function, or any other tool.

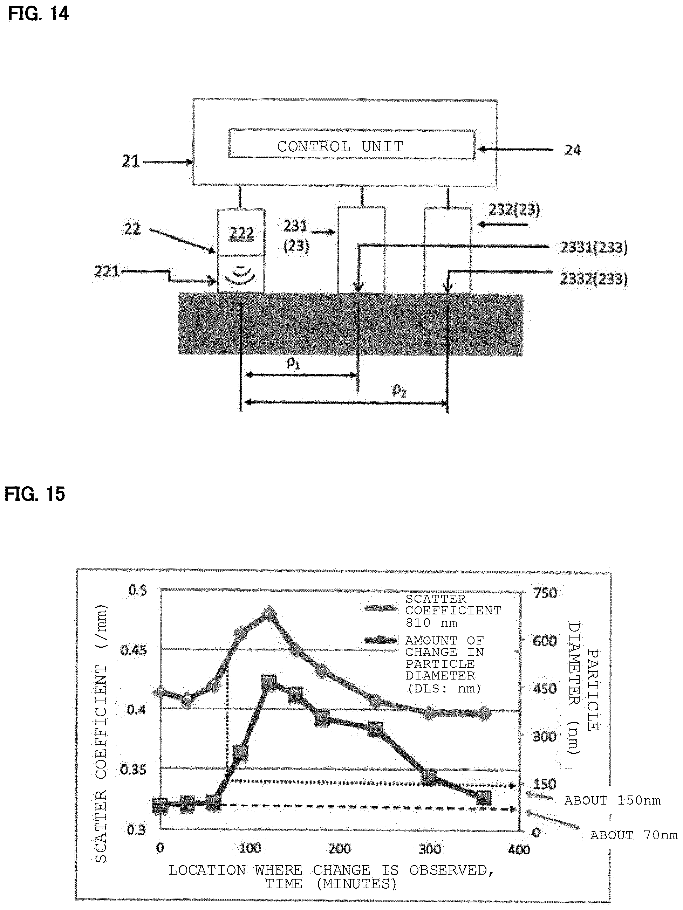

Figure 14 shows the configuration of an in-blood lipid concentration measurement device according to a second embodiment. The configuration of the lipid concentration measurement device according to the second embodiment has portions common to those of the configuration of the lipid concentration measurement device according to the first embodiment, and different portions will therefore be primarily described.

A lipid concentration measurement device 21 includes a light radiator 22, an optical intensity detector 23, and a control unit 24, as shown in Figure 14.

The configuration, action, and function of the light radiator 22 are the same as those of the light radiator 12 in the first embodiment.

The optical intensity detector 23 receives radiated light emitted from the living body toward the space outside the living body and detects the optical intensity of the radiated light. In a case where a plurality of optical intensity detectors 23 are used, the optical intensity detectors 23 are placed respectively at different distances from a radiation position 221 as a rough center. In the present embodiment, a first optical intensity detector 231 and a second optical intensity detector 232 are sequentially arranged linearly on the same surface at predetermined intervals from the radiation position 221, as shown in Figure 14. The optical intensity detectors 23 may each be a CCD, a CMOS device, or any other light receiving element.

In the present embodiment, the distance from the radiation position 221 to a first detection position 2331, where the first optical intensity detector 231 performs the detection, is called a first radiation detection distance $\rho1$, and the distance from the radiation position 221 to a second detection position 2332, where the second optical intensity detector 232 performs the detection, is called a second radiation detection distance p2, as shown in Figure 14.

In the case where a plurality of detection positions 233 are provided, the optical intensity detectors 23 are not necessarily arranged linearly as long as they are arranged respectively at different distances from the radiation position 221 as a rough center, and a circular arrangement, a wavy arrangement, a zigzag arrangement, or any other arrangement can be selected as appropriate. The first radiation detection distance p1 from the radiation position 221 to the detection position 231, the second radiation detection distance p2, and the gap between the detection positions 2331 and 2332 are not each limited to a fixed value and may instead each be continuously changed.

The optical intensity detector 23 may be so disposed as to face the light radiator 22, as in the first embodiment.

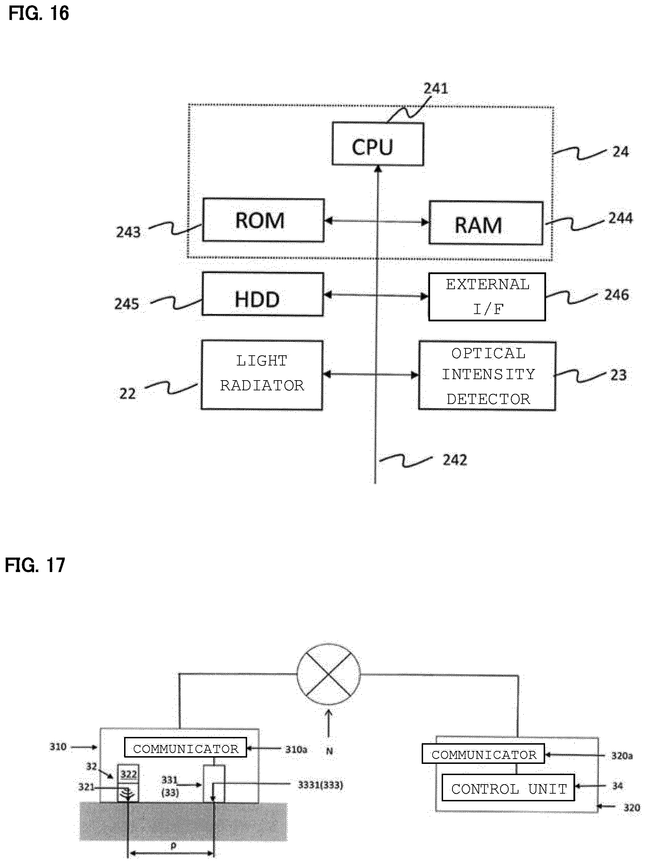

The configuration of a control system of the lipid concentration measurement device 21 according to the embodiment will next be described. Figure 16 is a block diagram of the lipid concentration measurement device 21 according to the embodiment. A CPU (central processing unit) 241, a ROM (read only memory) 243, a RAM (random access memory) 244, a storage 245, an external I/F (interface) 246, the light radiator 22, and the optical intensity detector 23 are connected to each other via a system bus 242. The CPU 241, the ROM 243, and the RAM 244 form the control unit (controller) 24.

The ROM 243 stores in advance a program executed by the CPU 241 and thresholds used by the CPU 241.

The RAM 244 has an area where the program executed by the CPU 241 is developed, a variety of memory areas, such as a work area where the program processes data, and other areas.

The storage 245 stores data, such as sensed and calculated optical intensities. The storage 245 may be an internal memory that stores information in a nonvolatile manner, such as an HDD (hard disk drive), a flash memory, and an SSD (solid-state drive).

The external I/F 246 is an interface for communication with an external device, for example, a client terminal (PC). The external I/F 246 only needs to be an interface that performs data communication with the external device and may, for example, be an instrument (such as USB memory) locally connected to the external device or a network interface for the communication via a network.

The control unit 24 calculates a scatter coefficient $\mu s'$ in the living body (including blood, skin, muscle, and other body parts, the same hereinafter) based on the optical intensity detected by the optical intensity detector 23.

The scatter coefficient $\mu s'$ in the embodiment is not limited to a digitized efficiency of a typical scatter process and includes a digitized influence of the scattering under a fixed condition in consideration of the scatter phenomenon.

A scatter coefficient calculator 24 includes two calculators, an optical intensity ratio calculator 241 and an optical intensity difference calculator 242, as shown in Figure 14.

The control unit 24 calculates the scatter coefficient $\mu s'$ based on each ratio between the optical intensities detected by the plurality of optical intensity detectors 23. The control unit 24 calculates the scatter coefficient $\mu s'$ based on a scatter phenomenon in which the radiated light attenuates due to the scattering as the distance to the detection position 233 increases.

In the embodiment, the light radiator 22 radiates continuous light having a predetermined optical intensity, and the scatter coefficient $\mu s'$ is calculated based on the ratio between an optical intensity $R(\rho1)$ detected by the first optical intensity detector 231 and an optical intensity R(p2) detected by the second optical intensity detector 232.

(Equation 1)

$$\mu s' = R(\rho1)/R(\rho2)$$

The control unit 24 calculates the scatter coefficient $\mu s'$ based on the difference between the optical intensities detected by the plurality of optical intensity detectors 23. The scatter coefficient $\mu s'$ is calculated based on the scatter phenomenon in which the radiated light attenuates due to the scattering as the distance to the detection position 233 increases, as in the optical intensity ratio calculation.

The control unit 24 calculates the scatter coefficient $\mu s'$ based on the difference between the optical intensity $R(\rho1)$ in the first detection position 2331 and the optical intensity R(p2) in the second detection position 2332.

(Equation 2)

$$\mu s' = R(\rho1) - R(\rho2)$$

The method in accordance with which the control unit 24 calculates the scatter coefficient μs' is not limited to each of the calculation methods described above.

The control unit 24 calculates the turbulent flow intensity I in the blood flow based on the calculated scatter coefficient μs'.

The turbulent flow intensity I in the blood flow in a blood vessel can be determined as follows: Variation σS in the scatter coefficient μs' in the sampling segment τ in noninvasive lipid measurement is determined by the expression below.

[Numerical expression 5]

$$\sigma S = \sqrt{S^2 + \overline{S}^2}$$

S : measured scatter coefficient in the measurement segment τ
$\overline{S}$ : average of the scatter coefficients in the measurement segment τ

The turbulent flow intensity I is determined by the expression below.

[Numerical expression 6]

$$I = \frac{\sigma S}{\overline{S}}$$

The control unit 24 calculates the in-blood lipid concentration based on the calculated turbulent flow intensity I. The method for calculating the lipid concentration has been described above.

In the embodiment, statistical data (or calibration curve) on the relationship between the turbulent flow intensity I and the amount of change in in-blood lipoprotein concentration is collected in advance, and the collected data is stored in the storage 245. The control unit 24 compares the measured turbulent flow intensity I with the stored statistical data or uses the calibration curve to calculate the amount of change in lipid concentration. The statistical data may be stored, for example, in the ROM 243.

In the aforementioned calculation of the turbulent flow intensity I, the turbulent flow intensity may be calculated when the amount of change in the average lipid particle diameter is greater than or equal to 80 nm. It can be ascertained that the scatter coefficient μs' increases when the amount of change in the average in-blood lipid particle diameter is greater than or equal to 80 nm, as shown in Figure 15. It is therefore preferable to measure a change in the turbulent flow when the amount of change in the lipid particle diameter is greater than or equal to 80 nm.

The average lipid particle diameter in Figure 15 (particle diameter along right vertical axis in Figure 15) has a baseline of about 70 nm. The particle diameter is an overall particle diameter of protein, lipid, and other substances that are in-plasma scatter components and the average particle diameter when hungry.

Lipid was loaded at the time of 0 minutes, and increases respectively in measured values of DLS and values measured by a lipid measurer at 90 minutes after the loading were observed. The particle diameter at which the change is observed is about 150 nm, and the amount of change in the average lipid particle diameter can be observed from the point corresponding to 80 nm.

The control unit 24 calculates the amount of change in the average in-blood lipoprotein particle diameter based on the amount of change in the calculated scatter coefficient μs'.

In the embodiment, statistical data on the relationship between the amount of change in the scatter coefficient μs' and the amount of change in average lipoprotein particle diameter is collected in advance, and the collected data is stored in the storage 245. The control unit 24 compares the amount of change in the scatter coefficient μs' with the stored statistical data to calculate the actual amount of change in average lipoprotein particle diameter.

For example, a calibration curve for calculating the amount of change in the average in-blood lipid particle diameter based on the amount of change in the scatter coefficient is created and stored in the storage 245. The control unit 24 uses the calibration curve to calculate an increased average particle diameter (Δ particle diameter) based on the difference between the scatter coefficient before the suspension in the blood and the scatter coefficient after the suspension (Δμs').

The format of the statistical data is not limited to a specific format. For example, the statistical data may be classified in terms of gender, body height, body weight, BMI, or any other factor and may be calculated by using a table, a graph, a function, or any other tool.

The average lipoprotein particle diameter used herein is the particle diameter expressed in the unit of nm. The average lipoprotein particle diameter ranges approximately from 80 to 1000 nm in the case of CM, from 30 to 80 nm in the case of VLDL, from 18 to 25 nm in the case of LDL, and from 7.5 to 10 nm in the case of HDL.

The average particle diameter used herein collectively expresses the following changes and conditions: That is, lipoprotein is classified into four types, and a change in the number of lipoprotein particles also affects the scattering. The number of four types of lipoprotein particles also slightly varies. That is, an increase or a decrease in the number of large particles changes the average particle diameter, and an increase or a decrease in the number of small particles also changes the average particle diameter. Therefore, an increase (or a decrease) in size of the large particles changes the average particle diameter, and an increase (or a decrease) in size of the small particles also changes the average particle diameter

The control unit 24 calculates the amount of change in average lipid particle diameter based on the amount of change in the scatter coefficient $\mu_s$'. The control unit 24 calculates the turbulent flow intensity I when the amount of change in average lipid particle diameter is greater than or equal to 80 nm.

A lipid concentration measurement device that is a third embodiment will be described below. The configuration of the lipid concentration measurement device according to the third embodiment has portions common to those of the configuration of the lipid concentration measurement device according to the first embodiment, and different portions will therefore be primarily described.

In the first embodiment described above, the configuration in which the light radiator 12, the optical intensity detector 13, and the control unit 14 are integrated with one another has been presented by way of example, but not necessarily. A light radiator 32, an optical intensity detector 33, and a control unit 34 may be configured as a system in which the light radiator 32, which radiates light, and the optical intensity detector 33 are configured as a user device and the control unit 34 is as a lipid concentration measurement device.

Figure 17 shows the configuration of the lipid concentration measurement device according to the third embodiment.

An in-blood lipid concentration measurement system 300 according to the embodiment is formed of a user device 310, which measures the optical intensity, and an in-blood lipid concentration measurement device 320, which calculates the lipid concentration based on the optical intensity. The user device 310 and the in-blood lipid concentration measurement device 320 are connected to each other over a wireless or wired communication network N.

The in-blood lipid concentration measurement device 320 is a device that carries out predetermined processes based on the optical intensity transmitted from the user device 310 to calculate the lipid concentration. Specifically, the in-blood lipid concentration measurement device 320 is formed, as appropriate of a personal computer or a server device depending on the number of required devices and the amount of data to be transmitted and received.

The user device 310 is a device that is carried by a user and is a standalone device in some cases or is incorporated in a mobile phone, a wristwatch, or any other product in other cases.

The user device 310 includes the light radiator 32, which radiates light, the optical intensity detector 33, and a communicator 310a. The communicator 310a transmits the optical intensity detected by the optical intensity detector 33. The action and function of the light radiator 32 and the optical intensity detector 33 are the same as those in the first embodiment.

The lipid concentration measurement device 320 includes a communicator 320a and the control unit 34. The communicator 320a receives the optical intensity transmitted from the communicator 310a over the wired or wireless network N and transmits the received optical intensity to the control unit 34. The action and function of the control unit 34 are the same as those of the control unit 14 in the first embodiment.

In the present embodiment, the optical intensity is transmitted from the user device 310 to the in-blood lipid concentration measurement device 320 over the network N, but not necessarily, and the user device 310 and the in-blood lipid concentration measurement device 320 may be directly connected to each other over no network N and may transmit the optical intensity over wired or wireless communication or any other means.

A lipid concentration measurement device that is a fourth embodiment will be described below. The configuration of the lipid concentration measurement device according to the fourth embodiment has portions common to those of the configuration of the lipid concentration measurement device according to the second embodiment, and different portions will therefore be primarily described.

In the second embodiment described above, the configuration in which the light radiator 22, the optical intensity detector 23, and the control unit 24 are integrated with one another has been presented by way of example, but not necessarily. A light radiator 42, an optical intensity detector 43, and a control unit 44 may be configured as a system in which the light radiator 42, which radiates light, and the optical intensity detector 43 are configured as a user device and the control unit 44 is configured as a lipid concentration measurement device.

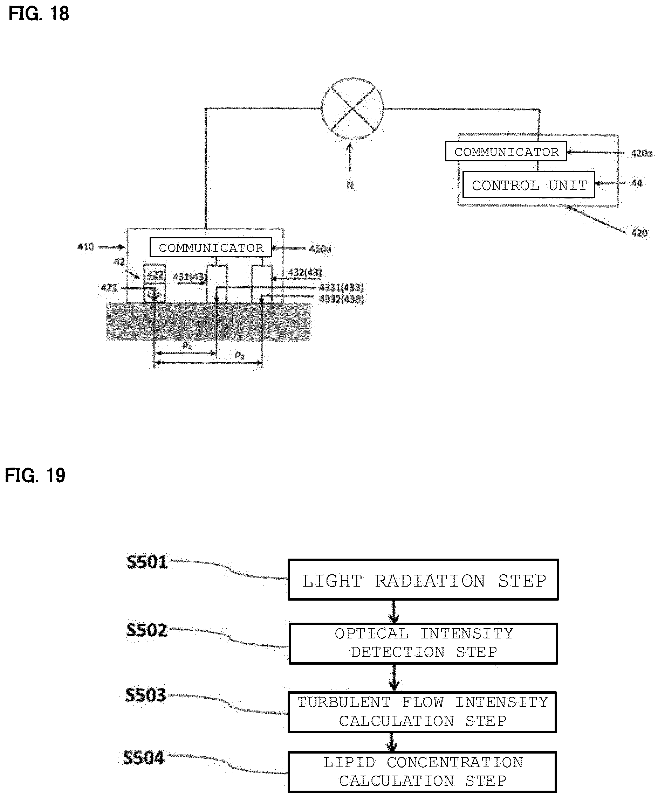

Figure 18 shows the configuration of the lipid concentration measurement system according to the fourth embodiment.

An in-blood lipid concentration measurement system 400 according to the present embodiment is formed of a user device 410, which measures the optical intensity, and an in-blood lipid concentration measurement device 420, which calculates the lipid concentration based on the optical intensity. The user device 410 and the in-blood lipid concentration measurement device 420 are connected to each other over the wireless or wired communication network N.

The in-blood lipid concentration measurement device 420 is a device that carries out predetermined processes based on the optical intensity transmitted from the user device 410 to calculate the lipid concentration. Specifically, the in-blood lipid concentration measurement device 420 is formed, as appropriate of a personal computer or a server device depending on the number of required devices and the amount of data to be transmitted and received.

The user device 410 is a device that is carried by a user and is a standalone device in some cases or is incorporated in a mobile phone, a wristwatch, or any other product in other cases.

The user device 410 includes the light radiator 42, which radiates light, the optical intensity detector 43, and a communicator 410a. The communicator 410a transmits the optical intensity detected by the optical intensity detector 43. The action and function of the light radiator 42 and the optical intensity detector 43 are the same as those in the second embodiment.

The lipid concentration measurement device 420 includes a communicator 420a and the control unit 44. The communicator 420a receives the optical intensity transmitted from the communicator 410a over the wired or wireless network N and transmits the received optical intensity to the control unit 44. The action and function of the control unit 44 are the same as those of the control unit 24 in the second embodiment.

In the present embodiment, the optical intensity is transmitted from the user device 410 to the in-blood lipid concentration measurement device 420 over the network N, but not necessarily, and the user device 410 and the in-blood lipid concentration measurement device 420 may be directly connected to each other over no network N and may transmit the optical intensity over wired or wireless communication or any other means.

A lipid concentration measurement method according to an embodiment will next be described. Figure 19 is a flowchart of the lipid concentration measurement method according to the embodiment. The lipid concentration measurement method according to the embodiment will be described by using the configuration of the lipid concentration measurement device according to the first embodiment, but the configuration of the device is not limited thereto.

The lipid concentration measurement device 11 according to the first embodiment performs the following lipid concentration measurement based on a program set in advance.

In a light radiation step (S501), the light radiator 12 is used to radiate continuous light to the radiation position 121.

In an optical intensity detection step (S502), the optical intensity detector 13 is used to detect the optical intensity in the detection position 131. The optical intensity detected in the detection position 131 is sent to a turbulent flow intensity calculation step.

In the turbulent flow intensity calculation step (S503), the turbulent flow intensity I in the blood flow is calculated based on the optical intensity detected in the optical intensity detection step. The method for calculating the turbulent flow intensity I has been described above.

In a lipid concentration calculation step (S504), the in-blood lipid concentration is calculated based on the turbulent flow intensity I. The method for calculating the lipid concentration has been described above. Instead, the amount of change in the average diameter of the lipid particles in the living body may be calculated based on the amount of change in the scatter coefficient, and when the amount of change in the average lipid particle diameter is greater than or equal to 80 nm, the turbulent flow intensity may be calculated.

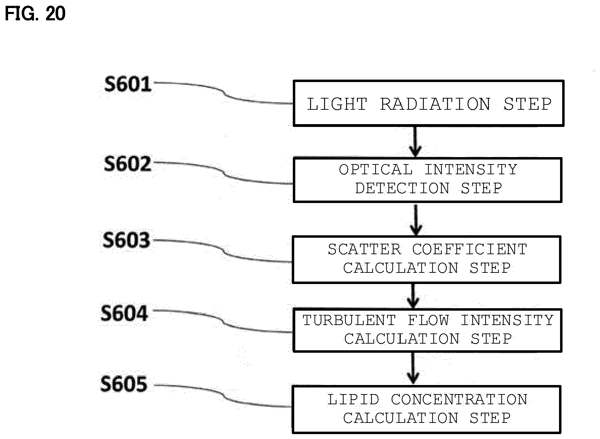

A lipid concentration measurement method according to another embodiment will next be described. Figure 20 is a flowchart of the lipid concentration measurement method according to the other embodiment. The lipid concentration measurement method according to the other embodiment will be described by using the configuration of the lipid concentration measurement device according to the second embodiment, but the configuration of the device is not limited thereto.

The lipid concentration measurement device 21 according to the second embodiment performs the following lipid concentration measurement based on a program set in advance.

In a light radiation step (S601), the light radiator 22 is used to radiate continuous light to the radiation position 221.

In an optical intensity detection step (S602), the first optical intensity detector 231 is used to detect the optical intensity in the first detection position 2331, and the second optical intensity detector 232 is used to detect the optical intensity in the second detection position 2332. The optical intensity detected in the first detection position 2331 and the optical intensity detected in the second detection position 2332 are sent to a scatter coefficient calculation step.

In the case where a plurality of detection positions 231 are provided, the optical intensity detectors 23 are not necessarily arranged linearly as long as they are arranged respectively at different distances from the radiation position 221 as a rough center, and a circular arrangement, a wavy arrangement, a zigzag arrangement, or any other arrangement can be selected as appropriate. The first radiation detection distance p1 from the radiation position 221 to the detection position 231, the second radiation detection distance p2, and the gap between the detection positions 2331 and 2332 are not each limited to a fixed value and may instead each be continuously changed.

In the scatter coefficient calculation step (S603), the optical intensity difference or the optical intensity ratio between the first detection position 2331 and the second detection position 2332 is calculated, and the scatter coefficient µs' is calculated based on the optical intensity difference or the optical intensity ratio. The calculated scatter coefficient µs' is sent to a turbulent flow intensity calculation step.

In the turbulent flow intensity calculation step (S604), the turbulent flow intensity I in the blood flow is calculated based on the scatter coefficient µs' calculated in the scatter coefficient calculation step. The method for calculating the turbulent flow intensity I has been described above.

In a lipid concentration calculation step (S605), the in-blood lipid concentration is calculated based on the turbulent flow intensity I. The method for calculating the lipid concentration has been described above. Instead, the amount of change in the average diameter of the lipid particles in the living body may be calculated based on the amount of change in the scatter coefficient, and when the amount of change in the average lipid particle diameter is greater than or equal to 80 nm, the turbulent flow intensity may be calculated.

As described above, according to the lipid concentration measurement device and the method therefor according to any of the embodiments, measurement of the turbulence of the blood flow can broaden the application range over which the device and the method are used and reduce individual differences in the lipid concentration measurement.

Reference Sings list

11: Lipid concentration measurement device
12: Light radiator
13: Optical intensity detector
14: Turbulent flow intensity calculator
15: Lipid concentration calculator

**Claims**

1. A lipid concentration measurement device, comprising:

   a light radiator that radiates light having a predetermined optical intensity to a biological body;
   an optical intensity detector that is located at a predetermined distance from the light radiator and detects an optical intensity of light emitted from the biological body; and
   a control unit that calculates a turbulent flow intensity in a blood flow based on the optical intensity and calculates a lipid concentration based on the turbulent flow intensity.

2. The lipid concentration measurement device according to claim 1, wherein a distance between the light radiator and the optical intensity detector is longer than or equal to 1.5 cm but smaller than or equal to 2.0 cm.

3. The lipid concentration measurement device according to claim 1 or 2, wherein the control unit calculates the turbulent flow intensity by using Numerical Expression 7 below,

   [Numerical expression 7]

   $$X = \frac{1}{T\bar{x}} \int_0^T x d\theta$$

   where T represents a measurement period, x represents a measured optical intensity, $\bar{x}$ represents an average of measured optical intensities, θ represents a period, and X represents a turbulent flow intensity I.

4. The lipid concentration measurement device according to claim 1 or 2, wherein the lipid is CM or VLDL.

5. A lipid concentration measurement device, comprising:

   a light radiator that radiates light having a predetermined optical intensity to a biological body;
   optical intensity detectors that are arranged at predetermined intervals from the light radiator, continuously from the light radiator, or in front of the light radiator and detect optical intensities of light emitted from the biological body; and
a control unit that calculates a light scatter coefficient in the biological body based on the optical intensities, calculates a turbulent flow intensity in a blood flow based on the scatter coefficient, and calculates a lipid concentration based on the turbulent flow intensity.

6. The lipid concentration measurement device according to claim 5, wherein the radiation position and a detection position where the optical intensity is detected are so provided as to be separate from each other by a predetermined radiation detection distance, and the optical intensity detectors detect optical intensities of backscattered light scattered by in-blood lipid.

7. The lipid concentration measurement device according to claim 5 or 6, wherein
the light radiator is a light source that outputs continuous light, the light source radiates the light, and the plurality of the optical intensity detectors placed respectively at different distances from a radiation position as a rough center detect optical intensities in respective detection positions, and
the control unit calculates a light scatter coefficient in the biological body based on a ratio or a difference among each of the optical intensities detected by each of the optical intensity detectors.

8. The lipid concentration measurement device according to any of claims 5 to 7, wherein the control unit calculates variation σS in the scatter coefficient μs' by using Numerical Expression 8 below and calculates the turbulent flow intensity based on the variation σS by using Numerical Expression 9 below,

[Numerical expression 8]

$$\sigma S = \sqrt{S^2 + \overline{S}^2}$$

where S represents a measured scatter coefficient in a measurement segment τ, $\overline{S}$ represents an average of the scatter coefficients in the measurement segment τ, and σS represents variation.

[Numerical expression 9]

$$I = \frac{\sigma S}{\overline{S}}$$

where σS represents variation, $\overline{S}$ represents the average of the scatter coefficients in the measurement segment τ, and I represents the turbulent flow intensity.

9. The lipid concentration measurement device according to any of claims 5 to 8, wherein the control unit
calculates an amount of change in an average lipid particle diameter in the biological body based on an amount of change in the scatter coefficient, and
calculates the turbulent flow intensity when the amount of change in the average lipid particle diameter is greater than or equal to 80 nm.

10. A lipid concentration measurement device communicably connected to a first device including a light radiator that radiates light having a predetermined optical intensity to a biological body, an optical intensity detector that is located at a predetermined distance from the light radiator and detects an optical intensity of light emitted from the biological body, and a communicator that transmits the optical intensity detected by the optical intensity detector, the lipid concentration measurement device comprising:
a control unit that calculates a turbulent flow intensity in a blood flow based on the optical intensity transmitted from the first device and calculates a lipid concentration based on the turbulent flow intensity.

11. A lipid concentration measurement device communicably connected to a first device including a light radiator that radiates light having a predetermined optical intensity to a biological body, optical intensity detectors that are arranged at predetermined intervals from the light radiator, continuously from the light radiator, or in front of the light radiator and detect optical intensities of light emitted from the biological body, and a communicator that transmits the optical intensities, the lipid concentration measurement device comprising:
a control unit that calculates a light scatter coefficient in the biological body based on the optical intensities transmitted from the first device, calculates a turbulent flow intensity in a blood flow based on the scatter coefficient, and calculates a lipid concentration based on the turbulent flow intensity.

12. A lipid concentration measurement method, comprising:

a light radiation step of radiating light having a predetermined optical intensity to a biological body;
an optical intensity detection step of detecting an optical intensity of light emitted from the biological body located at a predetermined distance from a position to which the light is radiated in the light radiation step;
a turbulent flow intensity calculation step of calculating a turbulent flow intensity in a blood flow based on the optical intensity; and
a lipid concentration calculation step of calculating a lipid concentration based on the turbulent flow intensity.

13. A lipid concentration measurement method, comprising:

a light radiation step of radiating light having a predetermined optical intensity to a biological body;
an optical intensity detection step of detecting optical intensities of light emitted from the biological body and measured at predetermined intervals from a position to which the light is radiated in the light radiation step, continuously from the position, or in front of the position;
a scatter coefficient calculation step of calculating a light scatter coefficient in the biological body based on the optical intensities;
a turbulent flow intensity calculation step of calculating a turbulent flow intensity in a blood flow based on the scatter coefficient; and
a lipid concentration calculation step of calculating a lipid concentration based on the turbulent flow intensity.

**FIG. 1**

A = SKIN LAYER
B = SKIN LAYER + BLOOD LAYER
C = SKIN LAYER + BLOOD LAYER + MUSCLE LAYER

**FIG. 2**

SCATTER COEFFICIENT IN HUNGRY STATE (FOREARM)

**FIG. 3**

AMPLITUDE OF BASE

TIME (5 MINUTES)

**FIG. 4**

FREQUENCY (Hz)

FIG. 5

FIG. 6

FIG. 7

FIG. 8

FIG. 9

FIG. 10

FIG. 11

SCATTERERS, SUCH AS LIPID AND THE LIKE

LIGHT RADIATOR

LIGHT RECEIVER

SUBJECT, SUCH AS BIOLOGICAL BODY

**FIG. 12**

SCATTERERS, SUCH AS LIPID AND THE LIKE

LIGHT RADIATOR          LIGHT RECEIVER

SUBJECT, SUCH AS BIOLOGICAL BODY

**FIG. 13**

EP 3 741 300 A1

FIG. 14

FIG. 15

# FIG. 16

# FIG. 17

FIG. 18

COMMUNICATOR — 420a
CONTROL UNIT — 44
420

COMMUNICATOR

N

410 →
42
421
422  431(43)  432(43)
4331(433)
4332(433)
COMMUNICATOR — 410a

ρ₁
ρ₂

FIG. 19

S501 ─ LIGHT RADIATION STEP

S502 ─ OPTICAL INTENSITY
DETECTION STEP

S503 ─ TURBULENT FLOW INTENSITY
CALCULATION STEP

S504 ─ LIPID CONCENTRATION
CALCULATION STEP

FIG. 20

S601 — LIGHT RADIATION STEP

S602 — OPTICAL INTENSITY DETECTION STEP

S603 — SCATTER COEFFICIENT CALCULATION STEP

S604 — TURBULENT FLOW INTENSITY CALCULATION STEP

S605 — LIPID CONCENTRATION CALCULATION STEP

## INTERNATIONAL SEARCH REPORT

| International application No. |
|---|
| PCT/JP2019/017746 |

**A. CLASSIFICATION OF SUBJECT MATTER**
Int.Cl. A61B5/1455(2006.01)i, G01N21/17(2006.01)i, G01N21/49(2006.01)i

According to International Patent Classification (IPC) or to both national classification and IPC

**B. FIELDS SEARCHED**

Minimum documentation searched (classification system followed by classification symbols)
Int.Cl. A61B5/06-5/22, G01N21/00-21/01, G01N21/17-21/61

Documentation searched other than minimum documentation to the extent that such documents are included in the fields searched
| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 10-258036 A (MATSUSHITA ELECTRIC INDUSTRIAL CO., LTD.) 29 September 1998, paragraphs [0001]-[0037], fig. 1-7 (Family: none) | 1-13 |
| A | JP 8-089500 A (I.S.S. (USA) INC.) 09 April 1996, paragraphs [0001]-[0075], fig. 1-7 & US 5497769 A, column 1, line 1 to column 9, line 60, fig. 1-5 & EP 663591 A1 | 1-13 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 July 2019 (18.07.2019) | 30 July 2019 (30.07.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/017746

C (Continuation).  DOCUMENTS CONSIDERED TO BE RELEVANT

| Category* | Citation of document, with indication, where appropriate, of the relevant passages | Relevant to claim No. |
|---|---|---|
| A | WO 2017/199492 A1 (MEDICAL PHOTONICS CO., LTD.) 23 November 2017, paragraphs [0001]-[0179], fig. 1-14 & EP 3459457 A1, paragraphs [0001]-[0178], fig. 1-13 & CN 109152558 A & KR 10-2019-0008357 A | 1-13 |
| A | WO 2017/141895 A1 (MEDICAL PHOTONICS CO., LTD.) 24 August 2017, paragraphs [0001]-[0133], fig. 1-19 & US 2019/0046091 A1, paragraphs [0001]-[0170], fig. 1-19 & EP 3417779 A1 & CN 108697388 A & KR 10-2018-0111956 A | 1-13 |
| A | WO 2017/119130 A1 (MITSUBISHI CHEMICAL HOLDINGS CORP.) 13 July 2017, paragraphs [0001]-[0044], fig. 1-8 (Family: none) | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014087825 A